# EUROPEAN PATENT SPECIFICATION

(11) **EP 2 641 573 B1**
(45) Date of publication and mention of the grant of the patent: **03.01.2018**
(21) Application number: 11841447.3
(22) Date of filing: 08.11.2011
(51) Int. Cl.: A61F 13/15, A61F 13/49

(54) **PRODUCTION METHOD FOR DISPOSABLE ITEM OF CLOTHING**
HERSTELLUNGSVERFAHREN FÜR EIN EINWEG-KLEIDUNGSSTÜCK
PROCÉDÉ DE PRODUCTION POUR ARTICLE D'HABILLEMENT JETABLE

(30) Priority: 18.11.2010 JP 2010257631
(43) Date of publication of application: 25.09.2013
(73) Proprietor: Zuiko Corporation, Osaka 566-0045 (JP)
(72) Inventor: UMEBAYASHI Toyoshi, Settu-Shi Osaka 566-0045 (JP)
(74) Representative: Schwabe - Sandmair - Marx
(86) International application number: PCT/JP2011/075692
(87) International publication number: WO 2012/066975

(56) References cited:
- EP-A1- 1 813 234
- EP-A1- 2 087 869
- WO-A1-2008/115099
- JP-A- 1 038 377
- JP-A- 1 272 803
- JP-A- 8 080 570
- JP-A- 2002 046 204
- JP-A- 2008 264 525
- JP-A- 2009 273 922

## Description

### TECHNICAL FIELD

The present invention relates to a method for manufacturing a disposable worn article.

### BACKGROUND ART

A method for manufacturing a disposable worn article while carrying a sheet and a core in the longitudinal direction perpendicular to the girth direction has been proposed in the art (see the first patent document). In the manufacturing method disclosed in the first patent document, identified below, liquid-absorbing cores are placed intermittently between a continuous top sheet and a continuous back sheet, thereby forming a laminate, after which the laminate is cut into individual disposable worn articles.

The second patent document, identified below, also discloses an external sheet obtained by laminating together a stretchable web that can be stretched in the girth direction and a non-stretchable web. However, it fails to give any disclosure as to the stretchability in the longitudinal direction. It also fails to give any disclosure as to the manufacturing method.

### CITATION LIST

### PATENT LITERATURE

[First Patent Document] Japanese Laid-Open Patent Publication No. 1-162808 (FIG. 4)
[Second Patent Document] Japanese Laid-Open Patent Publication No. 10-99373 (Paragraphs 0008, 0009)

### SUMMARY OF INVENTION

While a tension is given in the carrying direction of the continuous sheet to be the external sheet, the tension causes the continuous sheet to stretch in the carrying direction. This results in necking, where the continuous sheet shrinks in the girth direction perpendicular to the carrying direction, causing the finishing precision (accuracy of finishing) of the worn article to lower.

The amount of stretch in the carrying direction varies along with variation in the tension. Therefore, misalignment easily occurs to the position of the leg holes and the absorbent body with respect to the continuous sheet, causing the finishing precision to lower.

Therefore, it is an object of the present invention to provide a method for manufacturing a disposable worn article, with which the cause for lowering the finishing precision of a worn article is reduced. method for manufacturing a disposable worn article including an absorbent body and an external sheet, the absorbent body including a liquid-permeable top sheet forming a skin-contact surface to be in contact with a wearer, a liquid-impermeable back sheet forming a non-skin-contact surface on an opposite side of the skin-contact surface, and a core sandwiched between the top sheet and the back sheet for absorbing body fluid, the absorbent body including a front portion, a crotch portion and a rear portion covering a front surface of a torso of the wearer, a crotch of the wearer and a rear surface of the torso of the wearer, respectively, and the external sheet being fastened to the back sheet of the absorbent body and projecting, in a girth direction of the wearer, from the absorbent body in the front portion and the rear portion of the absorbent body, the method including the steps of:
carrying, in a carrying direction perpendicular to the girth direction, a continuous sheet to be the external sheet that is stretchable in the girth direction and substantially less stretchable in the carrying direction, while the continuous sheet is continuous in the carrying direction;
placing the absorbent body on the continuous sheet while the front portion, the crotch portion and the rear portion are lined up in the carrying direction; and
severing the continuous sheet along a virtual cut-off line extending in the girth direction, thereby obtaining individual worn articles each having the absorbent body placed on the external sheet.

The present invention is defined in claim 1 and directed to a method for manufacturing a disposable worn article including an absorbent body and an external sheet, the absorbent body including a liquid-permeable top sheet forming a skin-contact surface to be in contact with a wearer, a liquid-impermeable back sheet forming a non-skin-contact surface on an opposite side of the skin-contact surface, and a core sandwiched between the top sheet and the back sheet for absorbing body fluid, the absorbent body including a front portion, a crotch portion and a rear portion covering a front surface of a torso of the wearer, a crotch of the wearer and a rear surface of the torso of the wearer, respectively, and the external sheet being fastened to the back sheet of the absorbent body and projecting, in a girth direction of the wearer, from the absorbent body in the front portion and the rear portion of the absorbent body, the method including the steps of:
carrying, in a carrying direction perpendicular to the girth direction, a continuous sheet to be the external sheet of the worn article that is stretchable in the girth direction and substantially less stretchable in the carrying direction, while the continuous sheet is continuous in the carrying direction;
successively cutting off severed sheets to be the external sheets from a tip portion of the continuous sheet in the carrying direction;
separating the adjacent severed sheets from each other with a predetermined interval between the severed sheets in the carrying direction; and
placing the absorbent body so that the absorbent body spans (bridges) between a pair of severed sheets separated from each other in the carrying direction.

In the present invention, since an external sheet that is substantially less stretchable in the carrying direction is used, the external sheet is unlikely to stretch in the carrying direction while carrying the external sheet. Therefore, necking described above is unlikely to occur, or positional misalignment is unlikely to occur for leg holes and absorbent bodies.

In the present invention, there may be an object interposed between the external sheet fastened to the back sheet and the back sheet.

### BRIEF DESCRIPTION OF DRAWINGS

FIG. **1A** is a plan view showing a worn article, unfolded, according to Example 1, and FIG. **1B** is a cross-sectional view taken along line IB-IB in FIG. **1A****.**
FIGS. **2A** and **2B** are plan views each showing Example 1 of the manufacturing method.
FIG. **3A** is a plan view showing a worn article, unfolded, according to Example 2, and FIG. **3B** is a cross-sectional view taken along line IIIB-IIIB in FIG. **3A****.**
FIG. **4** is a schematic plan/perspective view showing Example 2 of the manufacturing method.
FIG. **5A** is a plan view showing a worn article, unfolded, according to Embodiment 1 of the present invention, and FIG. **5B** is a cross-sectional view taken along line VB-VB in FIG. **5A****.**
FIGS. **6A** and **6B** are plan views each showing Embodiment 1 of the manufacturing method of the present invention.
FIG. **7A** is a plan view showing a worn article, unfolded, according to Embodiment 2 of the present invention, and FIG. **7B** is a cross-sectional view taken along line VIIB-VIIB in FIG. **7A****.**
FIG. **8** is a schematic plan/perspective view showing Embodiment 2 of the manufacturing method of the present invention.

### DESCRIPTION OF EXAMPLES AND EMBODIMENTS

According to an example, the method further includes the steps of carrying a continuous laminate to be a plurality of absorbent bodies in the carrying direction while the continuous laminate is continuous in the carrying direction; successively cutting off the absorbent bodies from a tip portion of the continuous laminate in the carrying direction; and separating the cut-off absorbent bodies from one another in the carrying direction of the absorbent bodies, wherein: in the placement step, the absorbent bodies are placed intermittently on the continuous sheet; and the continuous sheet is severed between the adjacent absorbent bodies.

In this case, since absorbent bodies are placed intermittently, the external sheet is stretchable in the girth direction in the girth area between the absorbent bodies. This improves the fit to the wearer.

In a preferred embodiment in the aspect of the present invention, each portion to be the external sheet includes a first side portion projecting in the girth direction from the front portion of one external sheet and a second side portion projecting in the girth direction from the rear portion of another external sheet, the second side portion being continuous with the first side portion; and the method further includes the step of successively dividing the portion to be the external sheet along a virtual cut-off line extending in the girth direction between the first side portion and the second side portion in the portion to be the external sheet, and obtaining individual worn articles each having the absorbent body placed between the two divided external sheets.

In this case, portions to be external sheets are continuous with each other via an absorbent body therebetween, thus improving the handling.

On the other hand, the portions to be external sheets do not cover the entire absorbent body, but cover only a part of the absorbent body. Therefore, there is unlikely to be a waste of material.

In a more preferred embodiment in the aspect, in the step of carrying the continuous sheet, no tensile force in the girth direction is given to the continuous sheet.

In this case, necking is even more unlikely to occur in the continuous sheet.

In another preferred embodiment of the present invention, an elastic band for giving stretchability in the girth direction to a rear waist portion covering the rear surface of the torso of the wearer is placed on the rear waist portion of the external sheet, and an inner surface of the external sheet is exposed so as to be in contact with a skin of the wearer between the elastic band and the absorbent body; and
the method further includes the step of intermittently placing a plurality of elastic bands on the continuous sheet while no tensile force is given in the girth direction of the elastic bands.

In this case, because of the elastic band provided in the rear waist portion, the disposable worn article fits around the waist, thus improving the wearability. Between the elastic band and the absorbent body, the inner surface of the external sheet is exposed so as to be in contact with the skin. Therefore, the air permeability in the rear waist portion is maintained.

Since the elastic band is placed on the rear waist portion while no tensile force is given to the elastic band, no tensile force in the girth direction is given to the continuous sheet. Therefore, necking is unlikely to occur in the continuous sheet.

With an external sheet used in the present invention, typically, the stress when stretched by 10% in the longitudinal direction is 10 times or more that when stretched by 10% in the girth direction. A method for manufacturing a non-woven fabric forming such an external sheet is disclosed in Japanese Laid-Open Patent Publication No. 9-279453, the entire disclosure of which is herein incorporated by reference.

The present invention will be understood more clearly from the following description of preferred embodiments taken in conjunction with the accompanying drawings. Note however that the embodiments and the drawings are merely illustrative and should not be taken to define the scope of the present invention. The scope of the present invention shall be defined only by the appended claims. In the accompanying drawings, like reference numerals denote like components throughout the plurality of figures.

### [Example 1]

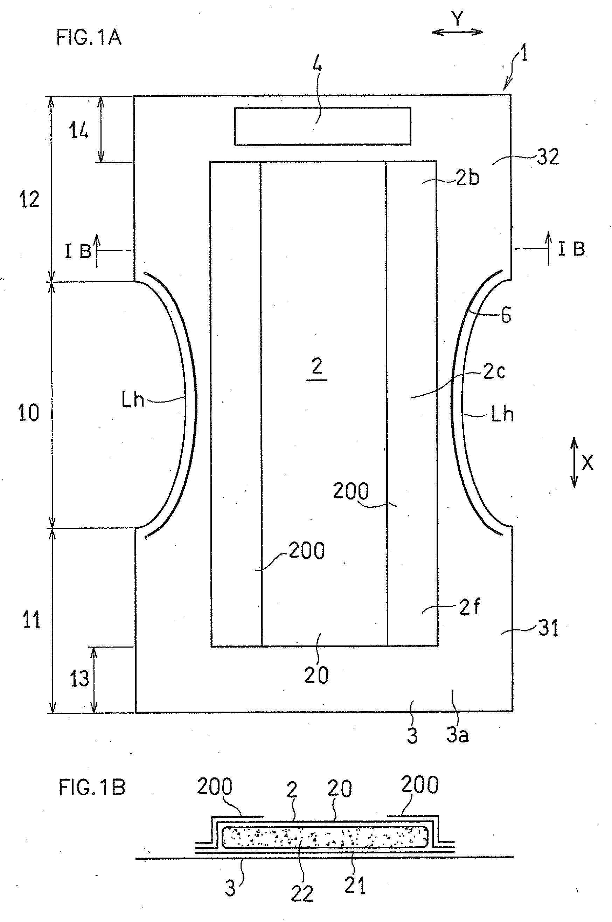

FIGS. **1A** to **2B** show Example 1.

### Diaper 1:

A diaper (an example of a worn article) 1 shown in FIG. **1A** is, for example, a pants-type diaper, and includes a crotch portion **10** covering the crotch of the wearer, a front torso portion 11 covering the abdominal side of the wearer, and a rear torso portion **12** covering the back side of the wearer.

The diaper 1 includes an absorbent body **2** and an external sheet **3.** The absorbent body **2** includes a front portion **2f,** a crotch portion **2c** and a rear portion **2b** covering the front surface of the torso of the wearer, the crotch of the wearer, and the rear surface of the torso of the wearer, respectively.

The external sheet **3** is formed by an air-permeable material (e.g., non-woven fabric). The external sheet **3** is formed by an anisotropic non-woven fabric that is stretchable in the girth direction Y and substantially less stretchable in the longitudinal direction **X** perpendicular to the girth direction **Y.**

As shown in FIG. **1B****,** the absorbent body **2** includes a liquid-permeable top sheet **20** and a liquid-impermeable back sheet **21,** with a core **22** for absorbing body fluid sandwiched therebetween.

The external sheet **3** is fastened to the back sheet **21** of the absorbent body **2,** and is projecting in the girth direction of the wearer from the absorbent body **2** in the front portion **2f** and the rear portion **2b** of the absorbent body **2.**

The core **22** is made of, for example, a pulp, a high-absorbent polymer, or the like.

A hydrophilic non-woven fabric may be placed as the core **22.** The top sheet **20** is made of a non-woven fabric covering the inner surface of the core **22,** forming a skin-contact surface to be in contact with the wearer, and the top sheet **20** may be provided with a three-dimensional non-woven fabric (cuffs) **200** for preventing side leaks. The back sheet **21** forms a non-skin-contact surface on the opposite side of the skin-contact surface, and covers the outer surface of the core **22.**

While the absorbent body **2** is placed on an inner surface **3a** of the external sheet **3,** as shown in FIG. **1A****,** it may be detachably fastened to the external sheet **3** via a touch fastener (not shown) therebetween.

A front waist portion **13** and a rear waist portion **14,** which are not covered by the absorbent body **2,** are formed in the front torso portion **11** and the rear torso portion **12** of the external sheet **3,** respectively.

The waist portions **13** and **14** of the external sheet **3** are not covered by the absorbent body **2.** Therefore, if a material having good air permeability (e.g., a non-woven fabric, or the like) is employed as the external sheet **3,** the air permeability of the waist portions **13** and **14** is improved, thereby preventing dampness.

The top sheet **20** and the back sheet **21** form the absorbent body **2** whose area is smaller than the external sheet **3.** Thus, the area of the top sheet **20** and the back sheet **21** is significantly reduced. Thus, there is no waste of material.

An elastic band **4** for giving stretchability in the girth direction **Y** to the rear waist portion **14** may be placed on the rear waist portion **14,** which covers the back side of the wearer. For example, a plurality of rubber threads, a rubber tape, a film, a material including a thermoplastic resin, or the like, may be employed as the elastic band **4.**

Therefore, when the diaper **1** is worn, the inner surface **3a** of the external sheet **3,** made of a non-woven fabric, is exposed so as to be in contact with the skin of the wearer in the front waist portion **13.** On the other hand, the inner surface **3a** of the external sheet **3** is in contact with the skin of the wearer between the elastic band **4** of the rear waist portion **14** and the absorbent body **2.**

In this example, because of the elastic band **4** provided in the rear waist portion **14** in addition to the shrinking force from the external sheet **3,** the disposable worn article fits around the waist, and the pants will not slip down when worn, thus improving the wearability. Here, between the elastic band **4** and the absorbent body **2,** the inner surface of the external sheet **3** is exposed so as to be in contact with the skin. Therefore, the air permeability in the rear waist portion **14** is maintained.

A first side portion **31** projecting in the girth direction **Y** from the front portion **2f** of the external sheet **3** and a second side portion **32** projecting in the girth direction **Y** from the rear portion **2b** of the external sheet **3** are welded together, forming the diaper **1** into a pants shape. Cut-outs to be leg holes **Lh,** through which legs of the wearer are passed, are formed between the first side portion **31** and the second side portion **32.**

In the vicinity of the opposite side edges in the girth direction **Y** of the absorbent body **2** corresponding to the leg holes **Lh** of the external sheet **3,** elastic members **6** made of rubber threads, for example, may be placed so that the external sheet **3** conforms around the legs of the wearer.

When the diaper **1** is worn, the diaper **1** fits around the waist of the wearer because of the shrinkage of the stretched elastic band **4** in addition to the shrinkage of the external sheet **3.** On the other hand, the elastic members **6** placed in the vicinity of the leg holes **Lh** shrink, whereby the diaper **1** fits around the legs of the wearer.

Note that the worn article may be a tape-type or fastener-type diaper, or the like, in which fastening members are provided so that the first side portion **31** and the second side portion **32** are not welded together but are fastened to each other by the fastening members.

Next, a method for manufacturing the diaper 1 will be described.

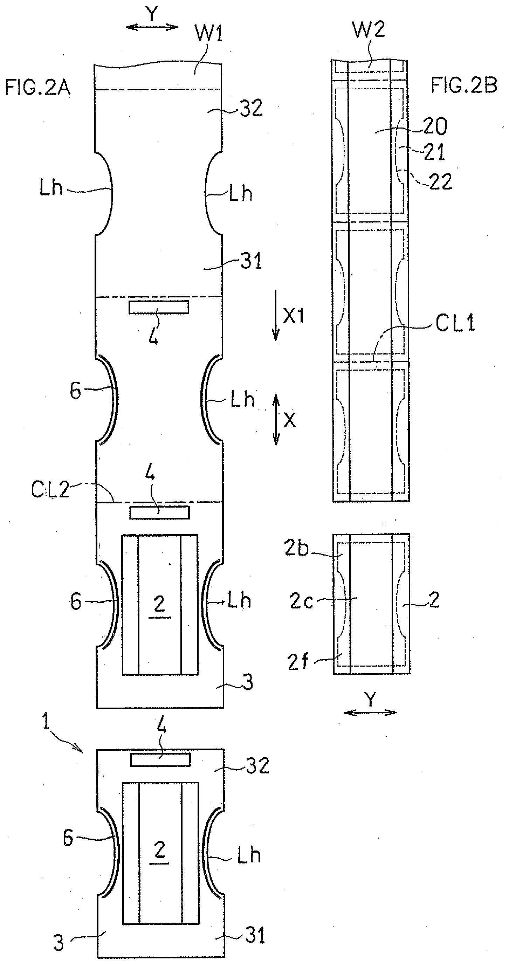

As shown in **FIG. 2A****,** a continuous sheet **W1** made of an anisotropic non-woven fabric is carried in the carrying direction **X1.** The continuous sheet **W1** includes a plurality of external sheets **3** of FIG. **1A** continuous with one another in the longitudinal direction **X** perpendicular to the girth direction **Y.** Therefore, the continuous sheet **W1** is stretchable in the girth direction **Y** and substantially less stretchable in the carrying direction **X1** perpendicular to the girth direction **Y.**

While the external sheet **3** and the continuous sheet **W1** are carried, a tensile force is given in the longitudinal direction **X** and the carrying direction **X1,** but no tensile force is given in the girth direction **Y.** That is, the continuous sheet **W1** is carried while being unstretched and shrunk in the girth direction **Y.**

Therefore, the external sheet **3** and the continuous sheet **W1** are essentially unstretchable either in the carrying direction **X1** (the longitudinal direction **X**) or the girth direction **Y,** thus avoiding necking.

### Trimming step:

While being carried, a portion of the continuous sheet **W1** is trimmed off in a die-cutting process so that a portion of the external sheet **3** is narrowed between the first and second side edges **31** and **32,** thus forming cut-outs to be the leg holes **Lh.**

After the trimming, the elastic bands **4** stretchable at least in the girth direction **Y** are placed with predetermined intervals. In this process, the elastic bands **4** are placed intermittently on the continuous sheet **W1** while no tensile force is given in the girth direction **Y** of the elastic band **4,** i.e., the elastic bands **4** are placed while being shrunk in the girth direction **Y.**

### Step of cutting off absorbent body 2:

On the other hand, as shown in FIG. **2B****,** a continuous laminate **W2** is carried in the carrying direction **X1.** The continuous laminate **W2** includes the absorbent bodies **2** of FIG. **1** continuous with one another in the longitudinal direction **X** perpendicular to the girth direction **Y.**

The continuous laminate **W2** includes the cores **22** sandwiched between the continuous top sheet **20** and the continuous back sheet **21.** As shown by broken lines of FIG. **2B****,** the cores **22** are placed intermittently between the top sheet **20** and the back sheet **21.**

The continuous laminate **W2** is successively cut, into the absorbent bodies **2,** along the first cut-off line **CL1** generally parallel to the girth direction **Y** at the tip portion of the continuous laminate **W2** in the carrying direction **X1.**

### Separation step:

After the severing (cutting off), as shown in FIG. **2B****,** the cut-off absorbent bodies **2** are separated from one another in the longitudinal direction **X.** For example, a re-pitching drum disclosed in Japanese Laid-Open Patent Publication No. 2006-212307 may be employed as the method for separating the absorbent bodies **2.**

### Step of placing absorbent body 2:

As shown in FIG. **2A****,** after the elastic members **6** made of rubber threads, for example, are intermittently placed on the continuous sheet **W1** along the longitudinal direction **X** in the vicinity of the leg holes **Lh,** the absorbent bodies **2** are intermittently placed on the continuous sheet **W1.**

In this process, the absorbent bodies **2** of FIG. **2B** are placed on the continuous sheet **W1** while the front portion **2f,** the crotch portion **2c** and the rear portion **2b** are lined up in the carrying direction **X1.** That is, the absorbent bodies **2** are placed on the continuous sheet **W1** so that the longitudinal direction **X** of the absorbent bodies **2** coincides with the longitudinal direction **X** of the external sheet **3.**

### Step of obtaining diaper 1:

Then, as shown in FIG. **2A****,** the continuous sheet **W1** is severed along the second cut-off line **CL2,** which is generally parallel to the girth direction **Y.** In this process, the continuous sheet **W1** is severed along the second cut-off line **CL2** between adjacent absorbent bodies **2.** Thus, individual diapers **1** are obtained, each including the absorbent body **2** placed on the external sheet **3.**

Note that the trimming step may be performed after the step of placing the absorbent bodies **2** or the step of obtaining individual diapers **1.**

### [Example 2]

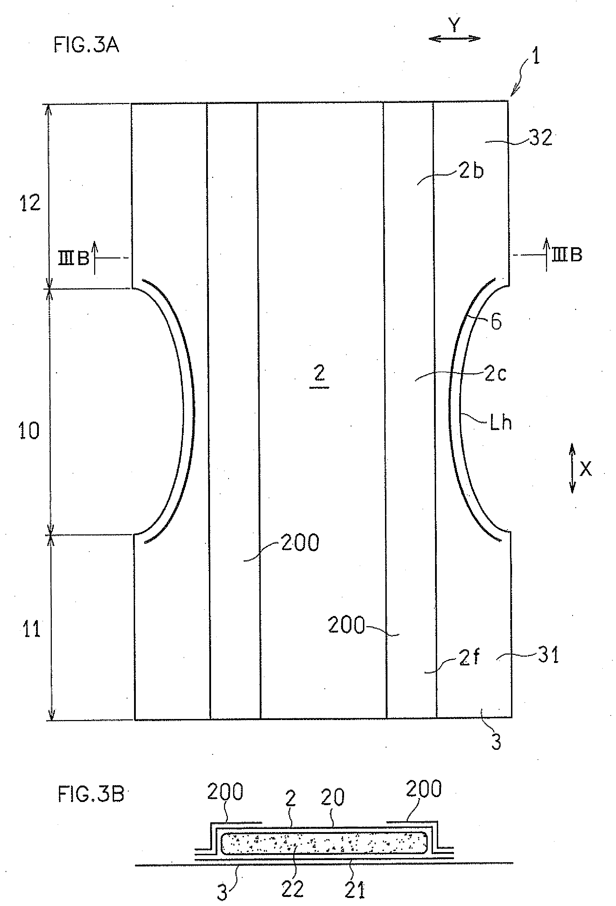

FIGS. **3A** to **4** showExample 2.

In Example 2, the absorbent body **2** of FIG. **3A** is provided extending from the front end to the rear end of the external sheet **3** in the longitudinal direction **X.** No elastic bands **4** are provided in Example 2.

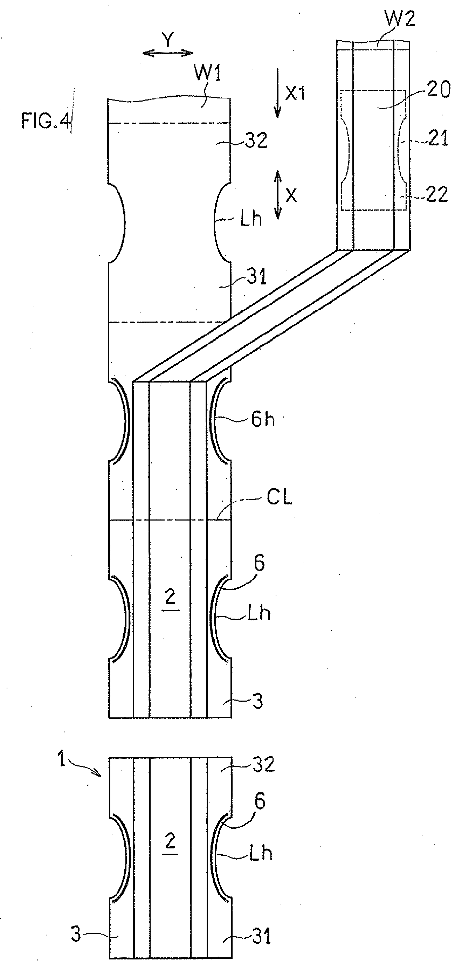

In this case, as shown in FIG. **4****,** after the continuous laminate **W2** is layered on the continuous sheet **W1,** the continuous sheet **W1** and the continuous laminate **W2** are successively severed along the cut-off line **CL.**

### [Embodiment 1]

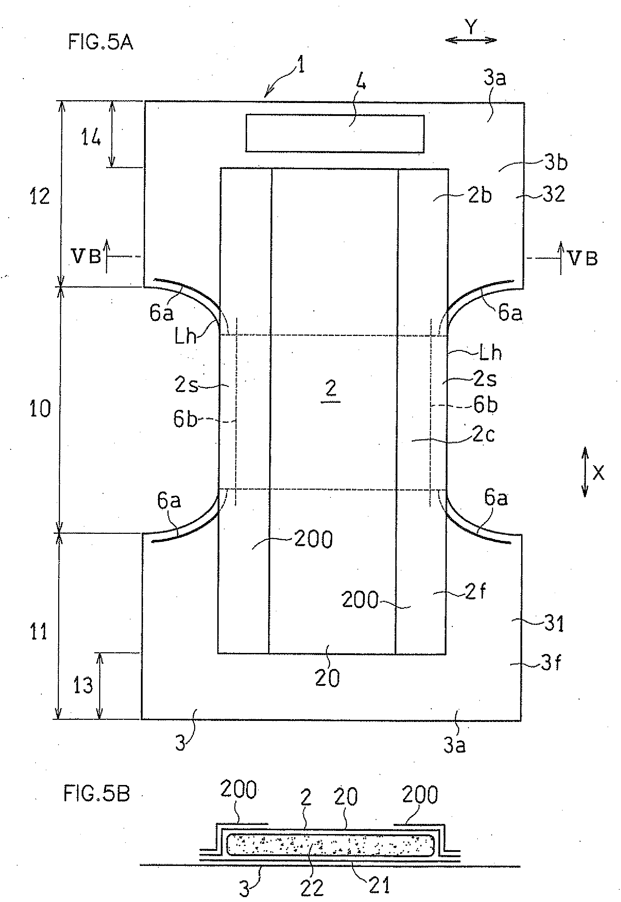

FIGS. **5A** to **6B** show Embodiment 1. In the Embodiment 1 to be described below, what are different from Example 1 described above will be mainly discussed.

### Diaper 1:

As shown in FIG. **5A****,** in the diaper 1 of the Embodiment 1, front and rear external sheets **3f** and **3b** are separate from each other in the crotch portion **10** between the front torso portion **11** and the rear torso portion **12.** The front and rear external sheets **3f** and **3b** are separated from each other in the longitudinal direction **X.** The absorbent body **2** is, for example, bonded with an adhesive and fastened to the pair of the front torso portion **11** and the rear torso portion **12** so as to span (bridge) between the front torso portion **11** and the rear torso portion **12.** Thus, the absorbent body **2** covers the crotch portion **10.**

The front waist portion **13** and the rear waist portion **14,** where the absorbent body **2** is absent, are formed in the front torso portion **11** and the rear torso portion **12,** respectively, of the external sheets **3f** and **3b,** which are divided in the front-rear direction. In the waist portions **13** and **14,** where the absorbent body **2** is absent, the external sheets **3f** and **3b** cover the waist of the wearer.

The elastic band **4** for giving stretchability in the girth direction **Y** to the waist portion **14** of the rear external sheet **3b** is placed on the rear external sheet **3b,** which covers the back side of the wearer, as in Example 1.

Therefore, the inner surface **3a** of the front waist portion **13** of the front external sheet **3f** is exposed so as to be in contact with the skin of the wearer, and the inner surface **3a** of the rear external sheet **3b** is exposed so as to be in contact with the skin of the wearer between the elastic band **4** of the rear waist portion **14** of the rear external sheet **3b** and the absorbent body **2.**

End portions of the external sheets **3f** and **3b,** opposing each other, are trimmed, thereby forming parts of the leg holes **Lh.** First elastic members **6a** made of rubber threads, for example, are placed along the end portions.

On the other hand, second elastic members **6b** and **6b** made of rubber threads, for example, are placed along opposite side edges **2s** and **2s** in the girth direction **Y** of the absorbent body **2** corresponding to the leg holes **Lh** for conformity around the legs of the wearer.

Note that the worn article may be a fastener-type diaper in which fastening members made of male touch fasteners (not shown), for example, are provided at opposite end portions of the rear external sheet **3b.**

The trimmed end portions of the external sheets **3f** and **3b** and portions of the opposite side edges **2s** of the absorbent body **2** together form the leg holes **Lh,** and the first and second elastic members **6a** and **6b** are placed around the leg holes **Lh.**

Next, a method for manufacturing the diaper **1** will be described.

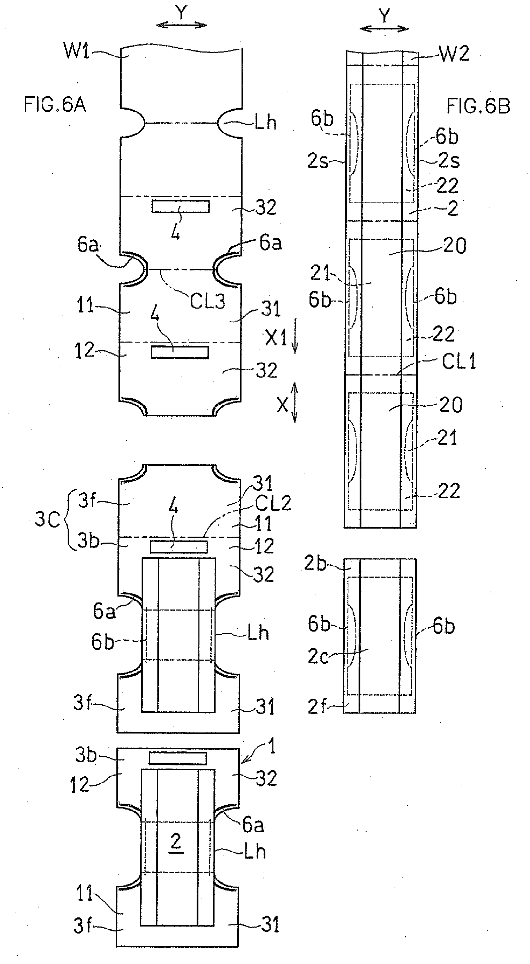

As shown in FIG. **6A****,** the continuous sheet **W1** made of an anisotropic non-woven fabric is carried in the carrying direction **X1.** The continuous sheet **W1** includes the external sheets **3f** and **3b** of FIG. **5** continuous with one another in the longitudinal direction **X** perpendicular to the girth direction **Y.** Therefore, the continuous sheet **W1** is stretchable in the girth direction **Y** and substantially less stretchable in the carrying direction **X1** perpendicular to the girth direction **Y.**

While the external sheets **3f** and **3b** and the continuous sheet **W1** are carried, a tensile force is given in the longitudinal direction **X** and the carrying direction **X1,** but no tensile force is given in the girth direction **Y.** That is, the continuous sheet **W1** is carried while being unstretched and shrunk in the girth direction **Y.**

Therefore, the external sheets **3f** and **3b** and the continuous sheet **W1** are essentially unstretchable either in the carrying direction **X1** (the longitudinal direction **X**) or the girth direction **Y,** thus avoiding necking.

While being carried, portions of the continuous sheet **W1** are trimmed off in a die-cutting process so that the external sheets **3f** and **3b** are narrowed between portions of the continuous sheet **W1** to be the first and second side portions **31** and **32.**

Then, as inExample 1, the elastic bands **4** are placed with predetermined intervals on the continuous sheet **W1.**

Prior to the die-cutting process, the first elastic members **6a** having a curved-arc shape are placed around the trimmed portions of the continuous sheet **W1** to be parts of the leg holes **Lh.**

Then, as shown in FIG. **6A****,** severed sheets **3C** are cut off from the continuous sheet **W1** along the third cut-off line **CL3.** The severed sheet **3C** is to be the rear and front external sheets **3b** and **3f** of adjacent diapers.

That is, the severed sheet **3C** includes one of the front torso portion 11 and the rear torso portion **12** to be the preceding diaper 1, and includes the other one of the front torso portion 11 and the rear torso portion 12 to be the next diaper 1 following the preceding diaper 1. That is, as shown by two-dot-chain line (the second cut-off line CL2) in FIG. 6A, in the severed sheet 3C, portions of the external sheets 3b and 3f to be end portions of worn articles have not been cut off from each other.

Then, the front and behind severed sheets 3C and 3C adjacent to each other are separated from each other with a predetermined interval therebetween in the longitudinal direction **X.**

### Step of cutting off absorbent body 2:

On the other hand, the continuous laminate **W2** is carried as shown in FIG. **6B****.** In the continuous laminate **W2,** the second elastic members **6b** are placed intermittently on the opposite side portions **2s** in the girth direction **Y** of the absorbent body **2** to be the crotch portion.

The continuous laminate **W2** is successively cut, into the absorbent body **2,** along the first cut-off line **CL1** generally parallel to the girth direction **Y** at the tip portion of the continuous laminate **W2** in the carrying direction **X1.**

### Separation step:

After the severing, the cut-off absorbent bodies **2** are separated from one another in the longitudinal direction **X.**

### Step of placing absorbent body 2:

Then, as shown in FIG. **6A****,** the absorbent body **2** is provided so as to span (bridge) between a pair of severed sheets **3C** and **3C** separated from each other. The absorbent body **2** is placed so as to span between the front torso portion **11** side and the rear torso portion **12** side of the pair of severed sheets **3C** and **3C.**

### Step of severing severed sheet 3C:

After the placement of the absorbent body **2,** the severed sheet **3C** is severed along the second cut-off line **CL2** to be divided into the front external sheet **3f** including the front torso portion **11** and the rear external sheet **3b** including the rear torso portion **12,** thereby producing individual diapers **1.**

Note that before the placement of the absorbent body **2,** the severed sheet **3C** may be severed in advance along the second cut-off line **CL2.**

### [Embodiment 2]

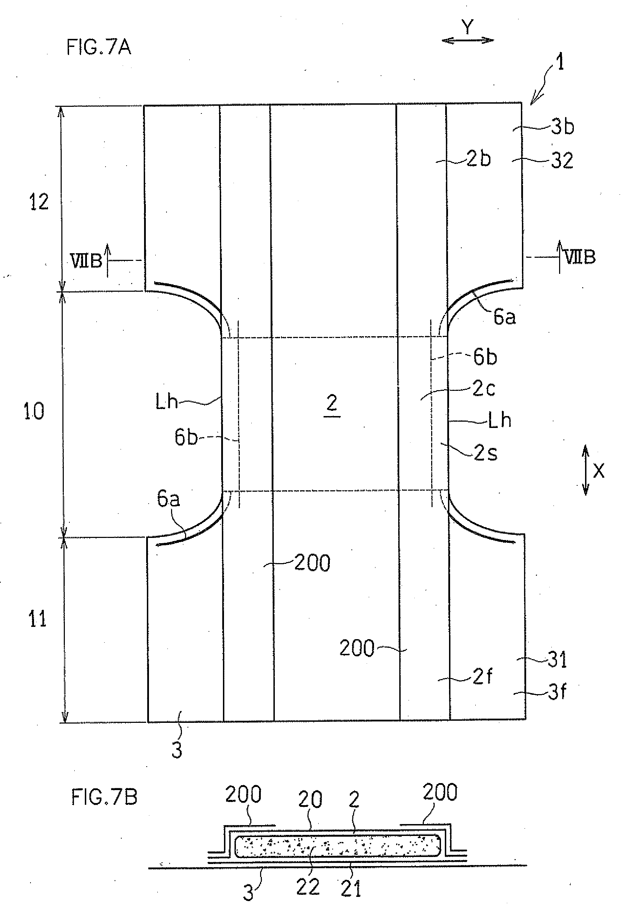

FIGS. **7A** to **8** show Embodiment 2.

In Embodiment 2, the absorbent body **2** of FIG. **7A** is provided extending from the front end to the rear end of the external sheet **3** in the longitudinal direction **X.** No elastic bands **4** are provided in Embodiment 4.

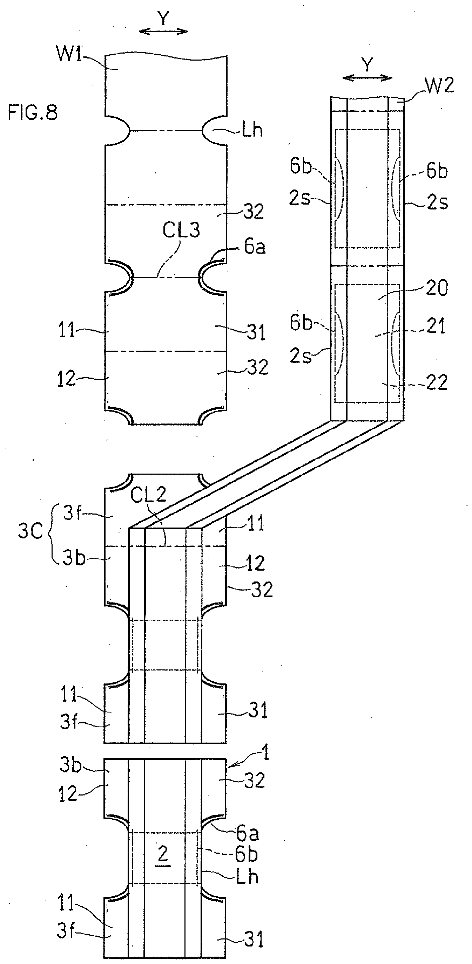

As shown in FIG. **8****,** in Embodiment 2, the severed sheets **3C,** which are portions to be the external sheets **3,** are successively cut along the third cut-off line **CL3** while the first side portion **31** projecting in the girth direction **Y** in the front torso portion **11** of one external sheet **3f** is continuous with the second side portion **32** projecting in the girth direction **Y** in the rear torso portion **12** of another external sheet **3b.** After the severing, the cut-off severed sheets **3C** are separated from each other in the longitudinal direction **X.**

The continuous laminate **W2** to be the absorbent bodies is placed, while being continuous, on portions to be external sheets and in areas therebetween. That is, the continuous laminate **W2** continuous in the longitudinal direction **X** is successively placed on the separated severed sheets **3C** and in areas therebetween.

Then, between the first side portion **31** and the second side portion **32** of the severed sheet **3C,** the severed sheet **3C** and the continuous laminate **W2** are successively severed along the virtual second cut-off line **CL2** extending in the girth direction, thereby obtaining individual worn articles each including the absorbent body **2** placed between two severed pieces of the external sheet.

Otherwise, the configurations and the manufacturing method of the diaper 1 of Embodiment 2 are similar to those of Embodiment 1 of FIGS. **5A** to **6****,** and therefore like elements are denoted by like reference numerals and will not be further described below.

While preferred embodiments have been described above with reference to the drawings, obvious variations and modifications will readily occur to those skilled in the art upon reading the present specification.

For example, the worn article manufactured by the present manufacturing method may be a tape-type or fastener-type diaper, or the like, in which the end portions of the front torso portion and the rear torso portion are not welded together but are fastened to each other by the fastening members.

### INDUSTRIAL APPLICABILITY

The present invention is applicable to methods for manufacturing disposable worn articles.

### REFERENCE SIGNS LIST

1: Diaper (an example of a worn article)
2: Absorbent body
3: External sheet
3C: Severed sheet
10: Crotch portion
20: Top sheet
21: Back sheet
22: Core
31: First side portion
32: Second side portion
W1: Continuous sheet
W2: Continuous laminate
X: Longitudinal direction
Y: Girth direction

## Claims

1. A method for manufacturing a disposable worn article (1) comprising an absorbent body (2) and an external sheet (3), the absorbent body (2) comprising a liquid-permeable top sheet (20) forming a skin-contact surface to be in contact with a wearer, a liquid-impermeable back sheet (21) forming a non-skin-contact surface on an opposite side of the skin-contact surface, and a core (22) sandwiched between the top sheet (20) and the back sheet (21) for absorbing body fluid, the absorbent body (2) including a front portion (2f), a crotch portion (10) and a rear portion (2b) covering a front surface of a torso of the wearer, a crotch of the wearer and a rear surface of the torso of the wearer, respectively, and the external sheet (3) being fastened to the back sheet (21) of the absorbent body (2) and projecting, in a girth direction of the wearer, from the absorbent body (2) in the front portion and the rear portion of the absorbent body (2), the method comprising the steps of:
carrying, in a carrying direction perpendicular to the girth direction, a continuous sheet (W1) to be the external sheet (3) of the worn article (1) that is stretchable in the girth direction and substantially less stretchable in the carrying direction, while the continuous sheet (W1) is continuous in the carrying direction;
successively cutting off severed sheets (3C) to be the external sheets (3b, 3f) from a tip portion of the continuous sheet (W1) in the carrying direction;
separating the adjacent severed sheets (3C) from each other with a predetermined interval between the severed sheets (3C) in the carrying direction; and
placing the absorbent body (2) so that the absorbent body (2) spans between a pair of severed sheets (3C) separated from each other in the carrying direction.

2. A method according to claim 1, wherein:
each severed sheet (3C) includes a first side portion (31) projecting in the girth direction from the front portion (2f) of one of the external sheets (3) and a second side portion (32) projecting in the girth direction from the rear portion (2b) of another one of the external sheets (3), the second side portion (32) being continuous with the first side portion (31); and
the method further comprises the step of successively dividing the severed sheets (3C) along a virtual cut-off line extending in the girth direction between the first side portion (31) and the second side portion (32) in the portion to be the external sheets (3), and obtaining individual worn articles (1) each having the absorbent body (2) placed between the two divided external sheets (3).

3. A method according to claim 2, wherein:
in the step of carrying the continuous sheet (W1), no tensile force in the girth direction is given to the continuous sheet (W1).

4. A method according to claim 3, wherein:
an elastic band (4) for giving stretchability in the girth direction to a rear waist portion covering the rear surface of the torso of the wearer is placed on the rear waist portion of the external sheet (3), and an inner surface of the external sheet (3) is exposed so as to be in contact with a skin of the wearer between the elastic band (4) and the absorbent body (2); and
the method further comprises the step of intermittently placing a plurality of the elastic bands (4) on the continuous sheet (W1) while no tensile force is given in the girth direction of the elastic bands (4).

## Patentansprüche

1. Verfahren zum Herstellen eines wegwerfbaren getragenen Artikels (1), der einen absorbierenden Körper (2) und eine äußere Folie (3) umfasst, wobei der absorbierende Körper (2) eine flüssigkeitsdurchlässige obere Folie (20), die eine Hautkontaktfläche bildet, um mit einem Träger in Kontakt zu sein, eine flüssigkeitsundurchlässige hintere Folie (21), die eine Nicht-Hautkontaktfläche auf einer gegenüberliegenden Seite der Hautkontaktfläche bildet, und einen Kern (22) der zwischen die obere Folie (20) und die hintere Folie (21) zum Absorbieren von Körperflüssigkeit eingefügt ist, umfasst, wobei der absorbierende Körper (2) einen vorderen Teil (2f), einen Schrittteil (20) und einen hinteren Teil (2b) umfasst, die jeweils eine vordere Fläche eines Rumpfes des Trägers, einen Schritt des Trägers und eine hintere Fläche des Rumpfes des Trägers bedecken, und die äußere Folie (3) an der hinteren Folie (21) des absorbierenden Körpers (2) befestigt ist und in eine Richtung des Körperumfangs des Trägers von dem absorbierenden Körper (2) in dem vorderen Teil und dem hinteren Teil des absorbierenden Körpers (2) vorsteht, wobei das Verfahren die Schritte umfasst:
Tragen in einer Tragerichtung senkrecht zu der Richtung des Körperumfangs einer durchgängigen Folie (W1), die die äußere Folie (3) des getragenen Artikels (1) bilden soll, die in Richtung des Körperumfangs dehnbar ist und im Wesentlichen in der Tragerichtung weniger dehnbar ist, während die durchgängige Folie (W1) in der Tragerichtung durchgängig ist;
nacheinander Abschneiden von abgetrennten Folien (3C), die die äußeren Folien (3b, 3f) bilden sollen, von einem Spitzenteil der durchgängigen Folie (W1) in der Tragerichtung;
Trennen der angrenzenden abgetrennten Folien (3C) voneinander mit einem vorgegebenen Intervall zwischen den abgetrennten Folien (3C) in der Tragerichtung; und
Positionieren des absorbierenden Körpers (2), derart, dass sich der absorbierende Körper (2) zwischen einem Paar abgetrennter Folien (3C), die voneinander in der Tragerichtung getrennt sind, aufspannt.

2. Verfahren nach Anspruch 1, wobei:
jede abgetrennte Folie (3C) einen ersten Seitenteil (31), der in Richtung des Körperumfangs von dem vorderen Teil (2f) einer der äußeren Folien (3) vorsteht, und einen zweiten Seitenteil (32), der in Richtung des Körperumfangs von dem hinteren Teil (2b) einer anderen der äußeren Folien (3) vorsteht, enthält, wobei der zweite Seitenteil (32) mit dem ersten Seitenteil (31) zusammenhängt; und
wobei das Verfahren ferner den Schritt umfasst, die abgetrennten Folien (3C) entlang einer virtuellen Schnittlinie, die sich in Richtung des Körperumfangs zwischen dem ersten Seitenteil (31) und dem zweiten Seitenteil (32) erstreckt, in den Teil, der die äußeren Folien (3) bilden soll, nacheinander zu trennen und einzelne getragene Artikel (1) zu erhalten, die jeweils den absorbierenden Körper (2) besitzen, der zwischen den beiden getrennten äußeren Folien (3) positioniert ist.

3. Verfahren nach Anspruch 2, wobei:
in dem Schritt des Tragens der durchgängigen Folie (W1) keine Zugkraft in Richtung des Körperumfangs auf die durchgängige Folie (W1) ausgeübt wird.

4. Verfahren nach Anspruch 3, wobei:
ein elastisches Band (4) an einem hinteren Taillenteil, der die hintere Fläche des Rumpfes des Trägers bedeckt, auf dem hinteren Taillenteil der äußeren Folie (3) positioniert wird, um eine Dehnbarkeit in Richtung des Körperumfangs zu geben, und eine innere Fläche der äußeren Folie (3) freigelegt wird, so dass sie mit einer Haut des Trägers zwischen dem elastischen Band (4) und dem absorbierenden Körper (2) in Kontakt ist; und
das Verfahren ferner den Schritt umfasst, mehrere der elastischen Bänder (4) auf der durchgängigen Folie (W1) zu positionieren, während keine Zugkraft in Richtung des Körperumfangs der elastischen Bänder (4) ausgeübt wird.

## Revendications

1. Procédé de production d'un article d'habillement jetable (1) comprenant un corps absorbant (2) et une couche externe (3), le corps absorbant (2) comprenant une couche supérieure perméable aux liquides (20) formant une surface de contact avec la peau destinée à être en contact avec un porteur, une couche arrière (21) imperméable aux liquides formant une surface sans contact avec la peau sur un côté opposé à la surface de contact avec la peau, et un coeur (22) pris en sandwich entre la couche supérieure (20) et la couche arrière (21) pour absorber des fluides corporels, le corps absorbant (2) incluant une section avant (2f), une partie d'entrejambe (10), et une partie arrière (2b) couvrant respectivement une surface avant d'un torse du porteur, un entrejambe du porteur et une surface arrière du torse du porteur, et la couche extérieure (3) étant fixée à la couche arrière (21) du corps absorbant (2) et faisant saillie, dans la direction de la taille du porteur, à partir du corps absorbant (2) dans la partie avant et la partie arrière du corps absorbant (2), le procédé comprenant les étapes consistant à :
transporter, dans une direction de transport perpendiculaire à la direction de la taille, une couche continue (W1) destinée à servir de couche externe (3) de l'article d'habillement (1), qui est extensible dans la direction de la taille et substantiellement moins extensible dans la direction de transport, la couche continue (W1) étant continue dans la direction de transport ;
successivement couper des couches sectionnées (3C) destinées à servir de couches externes (3b, 3f) d'une partie d'extrémité de la couche continue (W1) dans la direction de transport ;
séparer les couches sectionnées (3C) adjacentes d'un intervalle prédéterminé entre les couches sectionnées (3C) dans la direction de transport ; et
placer le corps absorbant (2) de telle manière que le corps absorbant (2) enjambe l'intervalle entre une paire de couches sectionnées (3C) séparées dans la direction de transport.

2. Procédé selon la revendication 1, où :
chaque couche sectionnée (3C) inclut une première partie latérale (31) faisant saillie dans la direction de la taille à partir de la partie avant (2f) d'une des couches externes (3) et une seconde partie latérale (32) faisant saillie dans la direction de la taille depuis la partie arrière (2b) d'une autre des couches externes (3), la seconde partie latérale (32) s'étendant en continu avec la première partie latérale (31) ; et
le procédé comprend en outre l'étape consistant à successivement diviser les couches sectionnées (3C) le long d'une ligne de coupe imaginaire s'étendant dans la direction de la taille entre la première partie latérale (31) et la seconde partie latérale (32) dans la partie destinée à servir de couche externe (3), et obtenir des articles d'habillement distincts (1) comportant chacun le corps absorbant (2) entre les deux couches externes divisées (3).

3. Procédé selon la revendication 2, où :
lors de l'étape de transport de la couche continue (W1), aucune force de traction n'est exercée sur la couche continue (W1) dans la direction de la taille.

4. Procédé selon la revendication 3, où :
une bande élastique (4) servant à rendre extensible, dans la direction de la taille, une partie de taille arrière couvrant la surface arrière du torse du porteur, est placée sur la partie de taille arrière de la couche externe (3), et une surface intérieure de la couche externe (3) est exposée de manière à être en contact avec la peau du porteur entre la bande élastique (4) et le corps absorbant (2) ; et
le procédé comporte en outre l'étape consistant à placer de manière intermittente une pluralité de bandes élastiques (4) sur la couche continue (W1) sans exercer de force de traction dans la direction de la taille des bandes élastiques (4).
